# EUROPEAN PATENT SPECIFICATION

(11) **EP 1 952 801 B1**
(45) Date of publication and mention of the grant of the patent: **25.07.2018**
(21) Application number: 05857405.4
(22) Date of filing: 25.11.2005
(51) Int. Cl.: A61K 8/66, A61K 8/34, A61K 8/24, A61Q 11/00

(54) **ORAL CAVITY CARE CURATIVE AND PROPHYLACTIC COMPOSITION**
KURATIVE UND PROPHYLAKTISCHE ZUSAMMENSETZUNG ZUR PFLEGE DER MUNDHÖHLE
COMPOSITION DE SOINS ET DE PREVENTION POUR LA CAVITE BUCCALE

(43) Date of publication of application: 06.08.2008
(73) Proprietor: "WDS" Company, Moscow 123592 (RU)
(72) Inventor: MANASHEROV, Tamaz Omarovich, Moscow, 119926 (RU); MATELO, Svetlana Konstantinovna, Moskovskaya obl., 143422 (RU); GROSSER, Alexandr Vladimirovich, Moskovskaya obl., 143300 (RU)
(74) Representative: Papula Oy
(86) International application number: PCT/RU2005/000601
(87) International publication number: WO 2007/061328

(56) References cited:
- WO-A1-98/02135
- DE-A1- 19 854 086
- GB-A- 2 289 841
- GB-A- 2 290 234
- RU-C1- 2 188 627
- RU-C1- 2 204 379
- US-B1- 6 379 654
- DATABASE MEDLINE [Online] US NATIONAL LIBRARY OF MEDICINE (NLM), BETHESDA, MD, US; March 1990 (1990-03), WÅLER S M ET AL: "[Xylitol, mechanisms of action and uses].", XP002737107, Database accession no. NLM2399120 & DEN NORSKE TANNLAEGEFORENINGS TIDENDE MAR 1990, vol. 100, no. 4, March 1990 (1990-03), pages 140-143, ISSN: 0029-2303
- MAURER H R: "Bromelain: biochemistry, pharmacology and medical use", CMLS CELLULAR AND MOLECULAR LIFE SCIENCES, BIRKHAUSER VERLAG, HEIDELBERG, DE, vol. 58, no. 9, 1 August 2001 (2001-08-01) , pages 1234-1245, XP002995539, ISSN: 1420-682X, DOI: 10.1007/PL00000936
- 'Zubnaya pasta na travyanoi osnove SanSmail, katalog' SANRAIDER, [Online] 30 May 2005, XP008185916 Retrieved from the Internet: <URL:http://www.shop.alfafarm/RU/product_in fo.php>products_id=112>

## Description

### Field of application

The invention relates to stomatology and perfume industry in particular to therapeutic and prophylactic formulation for oral cavity care. The invention refers to oral hygiene means and can be applied for preparation of medical and prophylactic tooth-pastes, gels, liquids for oral care as well as other compositions like chewing gums, jellies, etc.

### Background of invention

There is known formulation for dental diseases and periodontium prevention comprising: sodium fluoride or sodium monofluorophosphate, sodium carboxymethylcellulose, titanium dioxide, sodium saccharate, sorbitol or glycerine, silica, food dye, vaseline oil, perfume and water, camomile extract, panthenol, calcium glycerophosphate, and polyvinylpyrrolidone. (RU C1 Nº 2188626, A61K7/16, 2002)

There is known composition aimed at remineralization of demineralized teeth parts by applying non-astringent composition on them containing xylitol 10-20% and at least one compound of ion-fluorides enough to provide concentration of 150-1800 ppm. Sodium fluoride prevails in the group of such ion-fluorides (RU C1 Nº2092153, A61K7/16, 1997) GB2289841 discloses oral care compositions comprising bromelain, xylitol and an oxidising agent, preferably sodium chlorite.

In accordance with this known solution, compositions contain one or several organic surface-active components for better moisturizing, foaming and prophylactic effect, as well as for thorough dispergating of the composition in oral cavity. Organic surface-active material is anionic, non-ionic or ampholytic by its nature. As surface-active component there is used washing material which provides the composition with washing and foaming properties.

Other materials may be added into oral medications as well, like bleaching agents, cutouts, silicons, chlorophyll compounds, other anticalculus agents and/or ammonia containing materials such as urea, diammonium phosphate and its mixes.

As referring to this invention therapeutic means may be in various forms like paste, gel, rinse, tablet, chewing composition.

In real life it is not always possible to secure favourable environment for prophylaxis, and known hygiene means do not have positive influence on oral cavity processes since effectiveness of applied additives depends on the facility of its access to oral tissues. In particular it can be explained by the fact that biofilm forming on dental surface impedes mineral penetration into teeth enamel whereas mucuous membrane resorbs substances selectively.

In known inventions therapeutic effect of remineralization cannot be achieved because of soft dental accretions and bacterial deposit on dental surface and soft tissues of mouth cavity. Components contained in well-known formulations do not provide for its effective long-term removal since components which perform function of deposit elimination and stay for the period between repeated tooth-brushing, are absent. Therefore, bacterial deposit induces dental diseases and periodontium, hampers processes of either natural mineral metabolism in mouth cavity or the one caused by medications aimed at dental protection.

### Substance of invention

Oral cavity care curative and prophylactic composition prepared according to the invention consists in improved teeth cleaning, maintenance of dental tissues remineralization, inhibition of bacterial dental deposit formation for at least 10-12 hours as well as enhanced enamel resisitivity and reduced inflammatory phenomena in periodontum because dental deposit delay ensures dental enamel saturation with saliva mineral components and aid to reduce microbal load on periodontum tissues. Moreover, this formulation does not contain any synthetic antibacterial medications and coarse abrasives. The main purpose of this invention was to elaborate high-efficiency formulation for prophylaxis of stomatological diseases of teeth and soft tissues of oral cavity in which synergist medications could heighten effect of each other attaining high therapeutic effect and results mentioned above.

Oral cavity care curative and prophylactic composition containing active and inert components orally applicable involves bromelain in amount of 0.01-1 wt %, xylitol 1.5-20 wt % and anti-carious mineral additive 0.05-3.0 wt % of the list of active components.

At best bromelain content is 0.1 - 0.8 wt %, xylitol content - 2.2-18 wt %.

Bromelain is a group of high-molecular glycoproteins (H. R. Maurer, CMLS Cell. Mol. Life Sci., 58 (2001), pp.1234-1245). Bromelain is found in fruits juice and in plant stalks. Its composition includes 8 proteases providing protein hydrolysis in wide range of medium pH (3.0 - 8.0). Bromelain was found by professor Heinicke in 1950.

Bromelain enzyme ensures effective removal of bacterial deposit, possesses anti-inflammatory and immunocorrective action which is associated with both direct proteolytic action of the enzyme and regulatory effect of its utilization products (peptide fragments). The enzyme is actively resorbed by mucosa over the whole length of gastrointestinal tract. Due to the presence of protease inhibitors it is safe for viable tissues.

Bromelain is used for alleviating inflammatory processes in injuries, prevention of soft tissues edema, as well as for enhancement of their reconstitution after traumas and other injuries. On oral application, it reduces inflammation and edema, and accelerates tissues reparation processes. Due to non-viable protein cleavage bromelain accelerates wound and trophic ulcer repair, providing its detersion from necrotic masses. Bromelain possesses immunocorrective action which is used to facilitate inflammatory processes in traumas, prevent soft tissues edema, accelerates processes of tissue reparation which is associated with vascular permeability modification and its ability to influence metabolism of arachidonic acid.

Recently there has been actively studied role of bromelain in virus infections protection particularly influenza viruses protection (A and B) (Ivanova V.T. et al. / Vopr.Virusol. 2003 sep-oct; 48(5): 14-8).

Bromelain remains in oral cavity for a long time inhibiting new deposit, improves soft tissue state. This effect is achieved due to bromelain double influence: on the hand it reduces microbial load by inhibiting dental deposit; on the other hand it enhances anti-inflammatory effect.

The main function of xylitol lies in modulation of dental enamel permeability. Mechanism of its involvement in biochemical metabolism of streptococci is characterized as lethal synthesis, resulting in reduced activity of pathogenic microorganisms and improved conditions of oral cavity organs that contributes to dental remineralization. (Tanzer J.M. / Xylitol chewing gum and dental caries. // Int. Dent. J., 1995 Feb; 45 (1 Suppl 1):65-76). Xylitol heightens remineralizing potential since it contributes to involvement of calcium in dental enamel, inhibits dental deposit formation, reduces cariogenic potential of microflora. Furthermore being a sweetener, xylitol improves its taste properties; being a polyatomic alcohol, it acts as a water-retaining component.

Lack of soft dental accretions provides teeth with constant access of mineral components contained in tooth-paste and saliva. Securing long-term protection from dental deposit formation the formulation aids to prevent main stomatological diseases.

Concept of remineralization is part of ideology of the formulation invented. Remineralizing system is a combination of ingredients saturating enamel of healthy teeth with minerals and initial cariogenic damage centres.

For attaining remineralizing effect formulation contains anti-carious mineral additive. At best anti-carious mineral additive content is 0.2-2.5 wt % and there are used calcium glycerophosphate and inorganic or organic magnesium salt.

Tooth purified from deposit is more amenable to influence of calcium, phosphor and magnesium which can be introduced into therapeutic and prophylactic formulation. Calcium and phosphor are basic building elements of teeth enamel and take part in metabolic processes through the whole human life.

Additives are necessary since teeth need these elements in caries and in non-carious teeth affections to a greater extent.

Non-carious teeth affections are often connected with calcium metabolic imbalance and are caused by adverse influence of endogeneous character like thyroid, pancreal, germ glands abnormalities, gastrointestinal tract diseases etc.; by unfavourable external influence such as ionizing radiation, daily long (more that 6 hours) computer work; by hazard influence such as acid fumes, metal dust and their combinations; a range of negative ecological effects, which result in substantial reduction of mineral components in dental tissues and then in affection in the form of caries, erosion, cuneate defects, teeth abradability.

Thereby local appliance of therapeutic and prophylactic formulations containing phosphor-calcium additives makes it possible not only to prevent but to a certain degree makes up for the loss in case of disease.

Calcium glycerophosphate is a source of active phosphorus and calcium supply to teeth and periodontium tissues and facilitates process of mineralization, improves anti-carious effect of formulation, intensifies anabolic processes in tissues.

Magnesium (of inorganic or organic salts) is a structural component of teeth. Magnesium is incorporated in complex formulation as microelement, which is a cofactor for phosphatases ensuring incorporation of phosphates into solid teeth tissues. Under the influence of phosphates there takes place hydrolysis of glycerophosphate and its bioavailability increases correspondingly.

Since the risk of teeth demineralization is substantially lower in absence of solid dental deposit it is probable that formulation does not involve fluorides. Fluoride content in mouth care formulation is relevant if soft dental deposit produces organic acids during breakdown of easy-fermented carbohydrates. However, local fluoride action cannot be traced on teeth enamel maturation (for people aging over 20) even under this condition. It is shown in loss of its efficiency in caries prophylaxis.

At the same time it is admitted in proposed composition that as anti-carious mineral additive there are used sodium monofluorphosphate, potassium monofluorphosphate, calcium monofluorphosphate or magnesium monofluorphosphate in amount of 0.5-1.5 wt %. Optimum concentration is 0.8-1.1 wt %.

If Oral cavity care curative and prophylactic composition were a paste it involves following inert components, wt %:
Abrasive component - 5-40
Moisturizing component - 5-70
Gel-forming component - 0.5-2.5
At least one surface-active component - 0.5-3.0
At least one flavour filler - 0.5-2
At least one preservative - 0.01-0.5

If composition were a gel it contains following inert components, wt %:
Moisturizing component - 5-70
Gel-forming component - 0.5-3.5
At least one surface-active component - 0.5-3.0
At least one flavour filler - 0.5-2
At least one preservative - 0.01-0.5

If composition were liquid, it comprises following inert components, wt %:
Moisturizing component - 5-70
At least one surface-active component - 0.5-3.0
At least one flavour filler - 0.5-2
Water - the rest

If composition were a chewing gum it consists of following inert components:
Polymer base - 20-30
At least one polyatomic alcohol - 45-60
At least one flavour filler - 0.5-2

If formulation were a jelly it includes following inert components, wt %:
Fruit sugar - 20-50
At least one structure-forming agent - 1-3
At least one flavour filler - 0.2-1

If curative and prophylactic composition were prepared in the form of a paste as abrasive component there can be used one or several substances selected out of the group including calcium carbonate, dicalcium phosphate, silica, aluminium oxide, calcium pyrophosphate, sodium metaphosphate, polymethacrylate, magnesium carbonate.

If curative and prophylactic composition were a paste or gel as moisturizing component there can be used one or several substances out of the group containing sorbitol, glycerine, polyethilenglycole. As gel-forming component there may be used carboxymethylcellulose, hydroxyethylcellulose, xanthan gum, carrageenan, guar gum.

In the process of paste or gel preparing, function of preservatives may be performed by one or several substances selected out of the group consisting of methylparaben, propylparaben, butylparaben and its sodium salts, and phenoxyethanol, benzole acid, sodium benzoate.

In the process of preparing of paste, gel or rinse formulations, there may be used such surface-active components as sodium laurylsulfate, alkylamidobetaine, PEG-40 hydrogenated castor oil (polyoxiethylen(40) hydrogenised castor oil), polysorbate-20 (polyoxiethylen -sorbithan-monolauriate)

In either of numbered formulations as a flavour filler there may be used one or several substances of the following group:
Peppermint oil, spearmint oil, sage oil, skinleaf oil, citrus, eucalyptic, firry, anisic, clove oils,
Menthol, carvone, anethol, methyl salicylate,
Sweeteners - sodium saccharinate, lactose, maltose, aspartame, sodium cyclamate.

### Realization of invention

Examples of curative and prophylactic composition in the form of a tooth-paste are illustrated in table 1.

Tooth-paste is prepared in the following way.

Weigh 2/3 of glycerine quantity required. Add to it xanthum gum and calcium glycerophosphate. Mix to formation of homogeneous mass.

Heat the required quantity of water in measuring bin to 75-78 °C, and then feed water into mixer.

Add to water sodium saccharate, xylitol, parabens, magnesium chloride (or magnesium glycerophosphate - in example 3). Mix to formation of transparent solution.

Add glycerine suspension of components to the solution obtained. Mix to formation of homogeneous mass.

Degas and mix for 10 minutes to full deaeration of the mixture.

**Table 1**

| | Example 1, mass.% | Example 2, mass.% | Example 3, mass.% |
|---|---|---|---|
| Glycerine | 20 | 8 | 5 |
| Sorbitol | - | 16 | 20 |
| Xylitol | 10 | 14 | 12 |
| Silica | 22 | 25 | - |
| Dicalcium phosphate | - | - | 35 |
| Xanthum gum | 1.2 | 1.2 | 1.0 |
| Sodium monofluorphosphate | - | 1.0 | - |
| Calcium glycerophosphate | 0.9 | 0.5 | 1.2 |
| Magnesium chloride | 0.12 | - | 0.16 |
| Magnesium gl ycerophosphate | - | 0.16 | - |
| Bromelain | 0.4 | 0.9 | 0.5 |
| Sodium laurylsulfate | 1.4 | - | 0.8 |
| Alkylamidobetaine | - | 1.0 | 0.8 |
| Polyvinylpirrolidon | 0.8 | 1.2 | - |
| Titanium dioxide | 0.3 | 0.4 | 0.2 |
| Methylparaben | 0.24 | 0.3 | 0.2 |
| Propylparaben | 0.08 | 0.1 | 0.06 |
| Sodium saccharinate | 0.2 | 0.1 | 0.15 |
| Perfume component | 1 | 0.8 | 1.1 |
| Water | Up to 100% | Up to 100% | Up to 100% |

Add titanium dioxide into mixer.

Degas and mix for 10 minutes to full deaeration of the mixture.

Add silica into mixer.

Degas and mix for 30-40 minutes.

Homogenize tooth-paste with homogenizing pump for 15-20 minutes.

Cool tooth-paste down to 40-45 °C with mixing.

Add bromelain to the rest of glycerine and mix till formation of homogeneous mass.

Feed bromelain suspension in 1/3 glycerine into mixer and mix for 20 minutes till formation of homogeneous mass.

Add perfume and sodium laurylsulfate (or amidobetain in example 1).

Mix for 30 minutes till formation of homogeneous mass.

The prepared tooth-paste is packed into tubes made of polymer material.

Examples of curative and prophylactic compositions for mouth care in the form of tooth gel are shown in table 2.

**Table 2**

| Components | Example 1, mass.% | Example 2, mass.% | Example 3, mass.% |
|---|---|---|---|
| Glycerine | 15 | 5 | 19 |
| Sorbitol | - | 15 | - |
| Xylitol | 8 | 10 | 8.0 |
| Hydroxyethylcellulose | 2.1 | 2.0 | 1.9 |
| Sodium monofluorphosphate | - | - | 0.8 |
| Calcium glycerophosphate | 1.2 | 1.8 | 0.5 |
| Magnesium chloride | 0.18 | - | 0.12 |
| Magnesium glycerophosphate | - | 0.2 | - |
| Bromelain | 0.2 | 0.3 | 0.4 |
| Guar gum | 0.09 | 0.08 | 0.06 |
| PEG-40 hydrogenated castor oil | 0.8 | 0.9 | 1.2 |
| Methylparaben | 0.3 | 0.24 | 0.2 |
| Sodium saccharinate | - | 0.06 | 0.1 |
| Perfume component | 0.4 | 0.6 | 0.5 |
| Water | Up to 100% | Up to 100% | Up to 100% |

Composition in the form of gel is prepared as follows.

Heat water as required in measuring bin to 75-82 °C, then feed water into mixer

Add methylparaben, magnesium chloride (or magnesium glycerophosphate as in example 2), xylitol, sodium saccharate, sodium monofluorphosphate (like in example 3), sorbitol (as in example 2)
Mix for 20 minutes to formation of transparent solution
Prepare suspension of bromelain, hydroxyethylcellulose, guar gum, calcium glycerophosphate in glycerine separately
Cool water solution of the components to the temperature of 50-55 °C.

Add suspension of bromelain and mix for 10 minutes till formation of homogenious mass.

Heat PEG-40 hydrogenized castor oil separately to 50-55 °C, add perfume and mix till formation of homogenious mass for 10 minutes.

Add the mix obtained to the solution prepared first and mix till formation of homogenious gel for 20-30 minutes.

Cool the gel obtained to the temperature of 20-25 °C while mixing and pack into tubes made of polymer material.

Examples of curative and prophylactic formulation for oral care in the form of rinse are listed in table 3.

Composition in the form of rinse is prepared in the following way.
Heat water as required in measuring bin up to 75-82 °C and feed into mixer
Add sodium benzoate, benzoic acid, calcium glycerophosphate
Mix till formation of homogeneous solution for 30-40 minutes
Add magnesium chloride (or magnesium glycerophosphate as in example 2), xylitol, polyvynilpirrolidone, sodium saccharate, sodium monofluorphosphate (like in example 3), sorbitol (as in examples 1 and 3) at a temperature of 70-75 °C and mix for 30-40 minutes.

Prepare suspension of bromelain, hydroxyethylcellulose in glycerine separately (or propylene glycol as in example 2).

Add the suspension to the formulation obtained first cooled down to 50-55 °C and mix till formation of transparent solution for 20 minutes.

Heat PEG-40 hydrogenated castor oil up to 50-55 °C, add perfume, mix till formation of homogeneous mix for 10 minutes and add the mix obtained earlier at a temperature of 50-55 °C. Mix formulation till formation of transparent solution.

Add alkylamidobetain (or sodium laurylsulfate as referred to example 3), mix to formation of transparent solution for 20 minutes, which is cooled in the process of mixing down to 20-25 °C and ladled into bottles.

**Table 3**

| Components | Example 1, mass.% | Example 2, mass.% | Example 3, mass.% |
|---|---|---|---|
| Glycerine | 2 | - | 3 |
| Sorbitol | 4 | - | 3 |
| Propylene glycol | - | 8 | - |
| Xylitol | 2.2 | 2.8 | 3.0 |
| Hydroxyethylcellulose | 0.05 | 0.06 | 0.04 |
| Sodium monofluorphosphate | - | - | 0.3 |
| Calcium glycerophosphate | 0.1 | 0.15 | 0.06 |
| Magnesium chloride | 0.04 | - | 0.02 |
| Magnesium glycerophosphate | - | 0.08 | - |
| Bromelain | 0.02 | 0.05 | 0.03 |
| Sodium laurylsulfate | - | - | 0.6 |
| Alkylamidobetaine | 0.6 | 0.6 | - |
| Polyvinylpirrolidon | 1.0 | 1.2 | 1.6 |
| PEG-40 hydrogenated castor oil | 1.2 | 0.9 | 0.8 |
| Benzole acid | 0.1 | 0.12 | 0.15 |
| Sodium benzoate | 0.25 | 0.2 | 0.3 |
| Sodium saccharinate | 0.1 | 0.08 | 0.06 |
| Perfume component | 0.25 | 0.28 | 0.3 |
| Water | Up to 100% | Up to 100% | Up to 100% |

Examples of medical and prophylactic chewing gum preparation are demonstrated in table 4.

**Table 4**

| Components | Example 1, mass.% | Example 2, mass.% | Example 3, mass.% |
|---|---|---|---|
| Gum base | 22 | 30 | 20 |
| Mannitol | 15 | - | 10 |
| Crystalline sorbitol | 30 | 38 | 28 |
| Perfume component | 1 | 0,6 | 0,8 |
| Magnesium chloride | 0,05 | 0,07 | 0,09 |
| Calcium glycerophosphate | 0,4 | 0,5 | 0,6 |
| Xylitol | 4,7 | 6,58 | 8,46 |
| Bromelain | 0,25 | 0,35 | 0,45 |
| Lecitin | 0,1 | 0,05 | 0,2 |
| Sodium saccharinate | 0,1 | 0,05 | 0,08 |
| Glycerine | 1 | 1,5 | 2 |
| Calcium carbonate | - | 2 | - |
| Starch hydrolisate | 10 | 10 | 15 |
| Noncrystalisable sorbitol | 15,4 | 10,3 | 14,32 |

Composition in the form of chewing gum is prepared as follows.

Flux gum base at a temperature of 90-95 °C, feed into mixer equipped with Z-type paddles and cool down to the temperature of 80°C.

Feed lecitin, calcium carbonate, magnesium chloride, calcium glycerophosphate and mix for 10 minutes, add noncrystalisable sorbitol and mix for 10 minutes, add mannitol and xylitol and mix for 10 minutes, add crystallized sorbitol, sodium saccharrinate, starch hydrolisate and mix for 10 minutes.

Cool the mix to 55 °C, add perfume, earlier prepared suspension of bromelain in glycerine and mix for 20 minutes.

Draw and roll the mass, cut it into slabs and cubes of required sizes, dye to required humidity and pack.

Examples of medical and prophylactic jelly (marmalade) are illustrated in table 5.

**Table 5**

| Components | Mass.% |
|---|---|
| Apple sauce | 25 |
| Granulate sugar | 20 |
| Corn syrup | 5 |
| Agar | 1 |
| Flavouring essence | 0.2 |
| Glycerine | 0.5 |
| Calcium glycerophosphate | 0.25 |
| Magnesium chloride | 0.04 |
| Xylitol | 5 |
| Bromelain | 0.02 |
| Dye | 0.001 |
| Water | Up to 100% |

Composition in the form of jelly (marmalade) is made in the following way.

Boil marmalade masses.

Withdraw moisture surplus at a temperature of 105-107 °C. Initial humidity of sugar-apple mix is more than 45%, humidity of sugar-dextrose-agar syrup is 30-33%. Final humidity of marmalade mass ranges from 24 to 33% and depends on formula and variety of products.

Boil sugar-dextrose-agar syrup in coiled boiling column to humidity indication of 27-28%.

Add xylitol, mix and cool down to the temperature of 40-50 °C.

Add flavouring essences, calcium glycerophosphate, magnesium chloride and suspension of bromelain in glycerine into cooled syrup.

On condensing jellify syrup into forms.

Leave to stand, withdraw jelly, cut into segments and pack it.

The effectiveness of therapeutic and prophylactic tooth-pastes prepared in accordance with the proposed invention has been tested on group of volunteers in order to evaluate its hygienic and anti-inflammatory influence.

There were used composition of examples 1-3 from table 6 with condition of everyday double tooth-brushing for 2-3 minutes.

Clinical tooth-paste survey involved analysis of cleansing and anti-inflammatory effect, evaluation of probable allergenic and locally irritating action.

Studies have been conducted for three months by a blind scheme, with stomatologist-epidemiologist conducting dental survey not knowing to which group each patient belongs.

Clinical studies included 10 people aged from 27 to 42 years.

Before studies, dental status had been evaluated for all the participants. The clinical investigation consisted of 4 series of tooth-paste tests. Each series comprised preliminary two-week use of identical tooth-paste of the "Colgate maximum caries protection" type and 14-days testing of studied tooth-pastes samples, which were given out to test persons in a random order.

**Table 6**

| | Example 1, mass.% | Example 2, mass.% | Example 3, mass.% |
|---|---|---|---|
| Glycerine | 20 | 22 | 25 |
| Xylitol | 10 | 12 | 14 |
| Silica | 22 | 24 | 26 |
| Xanthan gum | 1,2 | 1,3 | 1,4 |
| Methylparaben | 0,2 | 0,24 | 0,3 |
| Propylparaben | 0,08 | 0,1 | 0,12 |
| Sodium saccharinate | 0,1 | 0,2 | 0,3 |
| Titanium dioxide | 0,2 | 0,3 | 0,4 |
| Perfume component | 0,7 | 0,8 | 1,0 |
| Bromelain | 0,1 | 0,3 | 0,7 |
| Sodium laurylsulfate | - | 1,3 | 1,4 |
| Alkylamidobetaine | 1,2 | - | - |
| Calcium glycerophosphate | 0,6 | 0,8 | 1 |
| Magnesium chloride | 0,08 | 0,12 | - |
| Magnesium glycerophosphate | - | - | 0,16 |
| Drinking water | Up to 100% | Up to 100% | Up to 100% |

The studies comprised:
- examination of solid and soft tissues of oral cavity: lips, tongue, hard and soft palate, teeth and gums;
- determination of hygienic state in oral cavity by PHP index (Podshadley A.G., Haley P., 1968);
- evaluation of periodontal tissues state by gingivitis index (IG) (Loe H., Silness J..1963) indicating localization and severity of gingivitis.

Examinations have been conducted 5 to 6 hours after teeth brushing.

Changes in hygienic index of studies participants are presented in Table 7.

**Table 7**

| Nos of formulations studied | Formulation 1 | Formulation 2 | Formulation 3 |
|---|---|---|---|
| Initial state (M±m) | 2,43±0,42 | 2,62±0,42 | 2,60±0,53 |
| Examination Nº2 (M±m) | 2,05±0,40 | 2,27±0,36 | 2,02±0,35 |
| Examination Nº3 (M±m) | 1,55±0,37 | 1,77±0,27 | 1,51±0,35 |
| Examination Nº4 (M±m) | 1,43±0,35 | 1,59±0,40 | 1,32±0,39 |
| Examination Nº5 (M±m) | 1,40±0,41 | 1,38±0,38 | 1,40±0,26 |
| Examination Nº6 (M±m) | 1,38±0,28 | 1,34±0,32 | 1,37±0,32 |
| Examination Nº7 (M±m) | 1,42±0,18 | 1,37±0,25 | 1,29±0,25 |
| Examination Nº8 14th day (M±m) | 1,45±0,16 | 1,36±0,26 | 1,12±0,21 |
| Effectiveness (%) | 40,3 | 48,1 | 56,9 |

Investigations of tooth-paste containing 0.7 % bromelain have demonstrated that the initial values of hygienic PHP index in studies' participants amounted to 2.60±0.53, corresponding to unsatisfactory level of oral cavity hygiene. Results of subsequent examinations have revealed reliable improvement in hygienic condition of oral cavity in studies' participants, with PHP index diminishing towards the end of investigations to 1.12±0.21 (p<0.02). Cleaning effectiveness of the tooth-paste over the period of use amounted to 56.9%.

Similar trend has been observed also in studies of cleaning properties of the tooth-paste containing bromelain 0.3%. During its use, value of hygienic index has reliably decreased from 2.62±0.42 to 1.36±0.26 (p < 0.02). Cleaning effectiveness of this sample amounted to 48.1%.

Studies of cleaning properties of the paste containing bromelain 0.1% have revealed that values of hygienic index decrease in a similar manner, but to a lesser extent. This index amounted to 40.3%.

Changes in gingivitis index in participants of the studies are listed in Table 8.

**Table 8**

| Group | Formulation 1 | Formulation 2 | Formulation 3 |
|---|---|---|---|
| Initial state (M±m) | 1,11±0, 11 | 1,16±0,21 | 1,13±0,12 |
| Examination Nº2 (M±m) | 0,98±0,07 | 0,99±0,15 | 1,01±0,08 |
| Examination Nº3 (M±m) | 0,93±0,08 | 0,96±0,14 | 0,89±0,19 |
| Examination Nº4 (M±m) | 0,88±0,12 | 0,89+0,1 2 | 0,70±0,14 |
| Examination Nº5 (M±m) | 0,84±0,13 | 0,82±0,15 | 0,83±0,12 |
| Examination Nº6 (M±m) | 0,81±0,11 | 0,81±0,12 | 0,94±0,11 |
| Examination Nº7 (M±m) | 0,77±0,14 | 0,75±0,11 | 0,95±0,09 |
| Examination Nº8 14th day (M±m) | 0,79±0,14 | 0,71±0,12 | 0,92±0,14 |
| Effectiveness (%) | 28,8 | 38,8 | 18,6 |

Initial state of periodontal tissues of persons participating in the IG index test corresponds to a mild or medium severity gingivitis. Values of gingivitis index at initial examinations: 1.13±0.12 in the first series, 1.16±0.21 in the second series, and 1.11±0.11 in the third one. Reduction in inflammatory events rate in periodontal tissues amounted to 18.6% when using the tooth-paste with maximum bromelain concentration; 38.8% when using the tooth-paste with bromelain concentration 0.3%; and 28.8%, when using the paste containing bromelain 0.1%, correspondingly.

By subjective estimate of the majority of participants, a substantial delay had been observed in apparent dental deposit formation on frontal teeth during period of use of the proposed tooth-paste formulation, as well as prolonged retention of teeth smoothness on lingual surfaces (in a number of reports, for 24 hours and more). Besides, those persons who clean regularly side teeth surfaces with a dental floss, have pointed out to a sharp decrease in the amount of dental deposits on side areas of teeth when using tooth-pastes with bromelain concentrations 0.3% and 0.7%, in comparison with that containing bromelain 0.1% and Colgate paste.

Of special merit is the fact that during wash-out period a substantial deterioration had been observed both of hygienic index and indices characterizing periodontium condition.

Evaluation of the proposed tooth-paste formulation effects on dental enamel condition was performed in comparison with a tooth-paste having sodium fluoride concentration 0.15% in terms of F⁻ (Blend-a-Med tooth-paste) by acid enamel biopsy. Studies had been conducted for one month, with weekly control of the index determined by procedure described below. Sampling had been performed on the intact surfaces of frontal teeth. Each test group included 30 people from among typical production users.

Acid enamel biopsy method according to V.K.Leontjev and V.A.Distel', comprising application of strictly determined quantity of demineralizing liquid on the enamel, its sampling after a certain period of time and subsequent determination of calcium content in acid demineralisate, allows to determine rate of acidic solubility of enamel. Quantitative analysis of calcium and phosphor content (in mcg/1) acidic biopsy material is performed by spectrophotometry.

Test results are presented in Table 9.

Acid enamel biopsy results indicate to the strengthening of dental enamel structure. Reduction in calcium and phosphorus recovery in biopsy material indicates to the increase in acid and chemical resistivity of enamel, testifying to its hardening. During four weeks of the studies, as a result of using the proposed tooth-paste formulation, calcium recovery with acid decreased by 20.53% with bromelain concentration 0.3%, and by 20.71% with bromelain concentration 0.1%, whereas corresponding indicator of test persons brushing teeth with Blend-a-Med paste amounted to 11.95 %, indicating to high remineralizing potential of the proposed tooth-paste formulation.

**Table 9**

| | Formulation 1 | Formulation 2 | Blen-a-med tooth paste |
|---|---|---|---|
| Before | Ca 113,25, P 56,1 | Ca 113,25; P 55,0 | Ca 91,2; P 46,0 |
| 1 week | Ca 112,25; P 50,5 | Ca 110,20; P 50,8 | Ca 90,0; P 42,5 |
| 2 week | Ca 92,8; P 47,7 | Ca 99,25; P 48,3 | Ca 85,2; P 40,5 |
| 1 month | Ca 89,7; P 44,9 | Ca 90,0; P 45,7 | Ca 80,3; P 39,2 |
| Reduction in enamel solubility | Ca 20,79; P 19,96 | Ca 20,53; P 16,91 | Ca 11,95; P 14,7 |

## Claims

1. Oral cavity care curative and prophylactic composition, containing active and inert components applicable orally is **characterized by** containing bromelain in amount of 0.01-1 wt % and xylitol in quantity of 1.5-20 wt % which provide for antideposit and anti-inflammatory effect, inhibition of dental deposit formation and improving quality of remineralization, and anti-carious mineral additive containing calcium glycerophosphate and inorganic or organic magnesium salt in amount of 0.05-3.0 wt %.

2. Composition according to claim 1 is **characterized by** bromelain content of 0.1-0.8 wt %.

3. Composition according to claim1 is **characterized by** xylitol content of 2.2-18 wt %.

4. Composition according to claim 1 is **characterized by** anti-carious mineral additive content of 0.2-2.5 wt%.

5. Composition according to claim 1 **characterized by** containing sodium monofluorphosphate, potassium monofluorphosphate, calcium monofluorphosphate, magnesium monofluorphosphate in quantity of 0.5-1.5 wt % as anti-carious mineral additive.

6. Composition according to claim 1 **characterized by** containing sodium monofluorphosphate, potassium monofluorphosphate, calcium monofluorphosphate, magnesium monofluorphosphate in quantity of 0.8-1.1 wt % as anti-carious mineral additive.

7. Composition according to claim 1 in the form of paste is **characterized by** containing following inert components, wt %:
Abrasive component - 5-40
Moisturizing component - 5-70
Gel-forming component - 0.5-2.5
At least one surface-active component - 0.5-3.0
At least one flavour filler - 0.5-2
At least one preservative - 0.01-0.5

8. Composition according to claim 1 in the form of gel contains following inert components, wt %:
Moisturizing component - 5-70
Gel-forming component - 0.5-2.5
At least one surface-active component - 0.5-3.0 At least one flavour filler - 0.5-2
At least one preservative - 0.01-0.5

9. Composition according to claim 1 in the form of liquid **characterized by** containing following inert components, wt %:
Moisturizing component - 5-70
At least one surface-active component - 0.5-3.0
At least one flavour filler - 0.5-2
Water - the rest

10. Composition according to claim 1 in the form of a chewing gum including the following inert components, wt %:
Polymer base - 20-30
At least one polyatomic alcohol - 45-60
At least one flavour filler - 0.5-2

11. Composition according to claim 1 in the form of jelly is notable for involving following inert components, wt %:
Fruit sugar - 20-50
At least one structure-forming agent - 1-3
At least one flavour filler - 0.2-1

12. Composition according to claim 1 in case of being a pastille **characterized by** containing following inert components, wt %:
At least one polyatomic alcohol - 40-60
At least one structure-forming agent - 2-15
At least one flavour filler - 0.5-2

13. Composition according to claim 7 **characterized by** containing as abrasive component one or several substances selected out of the group consisting of calcium carbonate, dicalcium phosphate, silica, aluminium oxide, calcium pyrophosphate, sodium metaphosphate, polymethacrylate, magnesium carbonate.

14. Composition according to claims 7 or 8 is **characterized by** containing as moisturing agent one or several substances selected out of the group including sorbitol, glycerine, polyethylenglycole.

15. Composition according to claims 7 or 8 is **characterized by** containing as gel-forming component one or several substances selected out of the group including carboxymethylcellulose, hydroxyethylcellulose, xanthan gum, carrageenan, guar gum.

16. Composition according to claims 7 or 8 or 9 is **characterized by** containing as surface-active agent one or several substances selected out of the group including sodium laurylsulfate, alkylamidobetaine, PEG-40 hydrogenated castor oil, polysorbate-20.

17. Formulation according to claims 7 -12 is **characterized by** containing as flavour filler one or several substances selected out of the group including:
Peppermint oil, spearmint oil, sage oil, skin leaf oil, citrus, eucalyptus, firry, anisic, clove oils
Menthol, carvone, anethol, methyl salicylate
Sweeteners - sodium saccharinate, lactose, maltose, aspartame, sodium cyclamate.

18. Composition according to claims 7 or 8 is **characterized by** containing as preservative one or several substances selected out of the group including methylparaben, propylparaben, butylparaben or its sodium salts, phenoxyethanol, benzoic acid, sodium benzoate.

## Patentansprüche

1. Kurative und prophylaktische Zusammensetzung zur Pflege der Mundhöhle, enthaltend oral verabreichbare aktive und inerte Komponenten, **dadurch gekennzeichnet, dass** sie Bromelain in einer Menge von 0,01 - 1 Gew.-% und Xylit in einer Menge von 1,5-20 Gew.-%, das ablagerungs- und entzündungshemmende Wirkung, Hemmung von dentaler Ablagerungsbildung und Verbesserung von Remineralisierungsqualität bereitstellt, und karieshemmendes Mineraladditiv, enthaltend Calciumglycerophosphat und anorganisches oder organisches Magnesiumsalz in einer Menge von 0,05-3,0 Gew.-% enthält.

2. Zusammensetzung nach Anspruch 1, **gekennzeichnet durch** einen Bromelain-Gehalt von 0,1- 0,8 Gew.-%.

3. Zusammensetzung nach Anspruch 1, **gekennzeichnet durch** einen Xylit-Gehalt von 2,2-18 Gew.-%.

4. Zusammensetzung nach Anspruch 1 **gekennzeichnet durch** einen karieshemmenden Mineraladditiv-Gehalt von 0,2-2,5 Gew.-%.

5. Zusammensetzung nach Anspruch 1, **dadurch gekennzeichnet, dass** sie Natriummonofluorphosphate, Kaliummonofluorphosphate, Calciummonofluorphosphat, Magnesiummonofluorphosphat in einer Menge von 0,5-1,5 Gew.-% als karieshemmendes Mineraladditiv enthält.

6. Zusammensetzung nach Anspruch 1 **dadurch gekennzeichnet, dass** sie Natriummonofluorphosphat, Kaliummonofluorphosphat, Calciummonofluorphosphat, Magnesiummonofluorphosphat in einer Menge von 0,8-1,1 Gew.-% als karieshemmendes Mineraladditiv enthält.

7. Zusammensetzung nach Anspruch 1 in der Form einer Paste, **dadurch gekennzeichnet, dass** sie folgende inerte Komponenten enthält, in Gew.-%:
Abrasive Komponente - 5-40
Feuchtigkeitsspendende Komponente - 5 -70
Gel-bildende Komponente- 0,5-2,5
Mindestens eine oberflächenaktive Komponente- 0,5-3,0 Mindestens einen Aromafüllstoff - 0,5-2
Mindestens einen Konservierungsstoff - 0,01-0,5

8. Zusammensetzung nach Anspruch 1 in der Form von Gel, enthaltend folgende inerte Komponenten, in Gew.-%:
Feuchtigkeitsspenden Komponente- 5-70
Gel-bildende Komponente - 0,5-2,5
Mindestens eine oberflächenaktive Komponente- 0,5-3,0
Mindestens einen Aromafüllstoff - 0,5-2
Mindestens einen Konservierungsstoff - 0,01-0,5

9. Zusammensetzung nach Anspruch 1 in der Form einer Flüssigkeit, **dadurch gekennzeichnet, dass** sie folgende inerte Komponenten enthält, in Gew.-%:
Feuchtigkeitsspendende Komponente- 5-70
Mindestens eine oberflächenaktive Komponente- 0,5-3,0
Mindestens einen Aromafüllstoff - 0,5-2
Wasser - als Rest

10. Zusammensetzung nach Anspruch 1 in der Form eines Kaugummis, der folgende inerte Komponenten einschließt, in Gew.-%:
Polymer-Grundstoff - 20-30
Mindestens einen mehratomigen Alkohol - 45-60
Mindestens einen Aromafüllstoff - 0,5-2

11. Zusammensetzung nach Anspruch 1 in der Form von Gelee, das insbesondere folgende inerte Komponenten einschließt, in Gew.-%:
Fruchtzucker - 20-50
Mindestens einen Strukturbildner - 1-3
Mindestens einen Aromafüllstoff - 0,2-1

12. Zusammensetzung nach Anspruch 1, falls es sich um eine Pastille handelt, **dadurch gekennzeichnet, dass** sie folgende inerte Komponenten enthält, in Gew.-%:
Mindestens einen mehratomigen Alkohol - 40-60
Mindestens einen Strukturbildner - 2-15
Mindestens einen Aromafüllstoff - 0,5-2

13. Zusammensetzung nach Anspruch 7, **dadurch gekennzeichnet, dass** sie als abrasive Komponente eine oder mehrere Substanzen enthält, ausgewählt aus der Gruppe bestehend aus Calciumcarbonat, Dicalciumphosphat, Silica, Aluminiumoxid, Calciumpyrophosphat, Natriummetaphosphat, Polymethacrylat, Magnesiumcarbonat.

14. Zusammensetzung nach Anspruch 7 oder 8, **dadurch gekennzeichnet, dass** sie als feuchtigkeitsspendende Komponente eine oder mehrere Substanzen enthält, ausgewählt aus der Gruppe, die Sorbit, Glycerin, Polyethylenglycol enthält.

15. Zusammensetzung nach Anspruch 7 oder 8, **dadurch gekennzeichnet, dass** sie als gelbildende Komponente eine oder mehrere Substanzen enthält, ausgewählt aus der Gruppe, die Carboxymethylcellulose, Hydroxyethylcellulose, Xanthan-Gummi, Carrageenan, Guar-Gummi enthält.

16. Zusammensetzung nach Anspruch 7 oder 8 oder 9, **dadurch gekennzeichnet, dass** sie als oberflächenaktives Mittel eine oder mehrere Substanzen enthält, ausgewählt aus der Gruppe, die Natriumlaurylsulfat, Alkylamidobetain, PEG-40-hydriertes Castoröl, Polysorbat-20 enthält.

17. Formulierung nach einem der Ansprüche 7-12, **dadurch gekennzeichnet, dass** sie als Aromafüllstoff eine oder mehrere Substanzen enthält, ausgewählt aus der Gruppe, umfassend:
Pfefferminzöl, Spearmintöl, Citrus-, Eucalyptus-, Guaven-Salbei-, Anis-, Nelkenöl, Menthol, Carvon, Anethol, Methylsalicylat, Süßungsmittel - Natriumsaccharinat, Lactose, Maltose, Aspartam, Natriumcyclamat.

18. Zusammensetzung nach Anspruch 7 oder 8, **dadurch gekennzeichnet, dass** sie als Konservierungsstoff eine oder mehrere Substanzen enthält, ausgewählt aus der Gruppe, die Methylparaben, Propylparaben, Butylparaben oder ihre Natriumsalze, Phenoxyethanol, Benzoesäure, Natriumbenzoate enthält.

## Revendications

1. Composition curative et prophylactique pour soins de la cavité buccale, contenant des composants actifs et inertes qui peuvent être appliqués par voie buccale, **caractérisée en ce qu'**elle contient de la bromélaïne en quantité de 0, 01 à 1 % en poids et du xylitol en quantité de 1,5 à 20 % en poids, qui assurent un effet anti-dépôts et anti-inflammatoire, l'inhibition de formation de dépôts dentaires et l'amélioration de la qualité de reminéralisation, ainsi qu'un additif minéral anticarie contenant du glycérophosphate de calcium et un sel de magnésium inorganique ou organique en quantité de 0,05 à 3,0 % en poids.

2. Composition selon la revendication 1, **caractérisée par** une teneur en bromélaïne de 0,1 à 0,8 % en poids.

3. Composition selon la revendication 1, **caractérisée par** une teneur en xylitol de 2,2 à 18 % en poids.

4. Composition selon la revendication 1, **caractérisée par** une teneur en additif minéral anticarie de 0,2 à 2,5 % en poids.

5. Composition selon la revendication 1, **caractérisée en ce qu'**elle contient du monofluorophosphate de sodium, du monofluorophosphate de potassium, du monofluorophosphate de calcium, du monofluorophosphate de magnésium en quantité de 0,5 à 1,5 % en poids comme additif anticarie.

6. Composition selon la revendication 1, **caractérisée en ce qu'**elle contient du monofluorophosphate de sodium, du monofluorophosphate de potassium, du monofluorophosphate de calcium, du monofluorophosphate de magnésium en quantité de 0, 8 à 1, 1 % en poids comme additif minéral anticarie.

7. Composition selon la revendication 1 sous la forme d'une pâte, **caractérisée en ce qu'**elle contient les composants inertes suivants en % en poids :
composant abrasif - 5 à 40,
composant humidifiant - 5 à 70,
composant formateur de gel - 0,5 à 2,5,
au moins un composant tensioactif - 0,5 à 3,0,
au moins une charge aromatisante - 0,5 à 2 et
au moins un conservateur - 0,01 à 0,5.

8. Composition selon la revendication 1 sous la forme d'un gel contenant les composants inertes suivants en % en poids :
composant humidifiant - 5 à 70,
composant formateur de gel - 0,5 à 2,5,
au moins un composant tensioactif - 0,5 à 3,0,
au moins une charge aromatisante - 0,5 à 2 et
au moins un conservateur - 0,01 à 0,5.

9. Composition selon la revendication 1 sous la forme d'un liquide, **caractérisée en ce qu'**elle contient les composants inertes suivants en % en poids :
composant humidifiant - 5 à 70,
au moins un composant tensioactif - 0,5 à 3,0,
au moins une charge aromatisante - 0,5 à 2 et
de l'eau - pour le restant.

10. Composition selon la revendication 1 sous la forme d'une gomme à mâcher comprenant les composants inertes suivants en % en poids :
base polymère - 20 à 30,
au moins un alcool polyvalent - 45 à 60 et
au moins une charge aromatisante - 0,5 à 2.

11. Composition selon la revendication 1 sous la forme d'une gelée qui est connue pour impliquer les composants inertes suivants en % en poids :
sucre de fruit - 20 à 50,
au moins un agent formateur de structure - 1 à 3 et
au moins une charge aromatisante - 0,2 à 1.

12. Composition selon la revendication 1 dans le cas de la formation d'une pastille, **caractérisée en ce qu'**elle contient les composants inertes suivants en % en poids :
au moins un alcool polyvalent - 40 à 60,
au moins un agent formateur de structure - 2 à 15 et
au moins une charge aromatisante - 0,5 à 2.

13. Composition selon la revendication 7, **caractérisée en ce qu'**elle contient comme composant abrasif une ou plusieurs substances choisies dans le groupe constitué du carbonate de calcium, du phosphate dicalcique, de la silice, de l'oxyde d'aluminium, du pyrophosphate de calcium, du métaphosphate de sodium, d'un polyméthacrylate, du carbonate de magnésium.

14. Composition selon les revendications 7 ou 8, **caractérisée en ce qu'**elle contient comme agent humidifiant une ou plusieurs substances choisies dans le groupe comprenant le sorbitol, la glycérine, des polyéthylèneglycols.

15. Composition selon les revendications 7 ou 8, **caractérisée en ce qu'**elle contient comme composant formateur de gel une ou plusieurs substances choisies dans le groupe comprenant la carboxyméthylcellulose, l'hydroxyéthylcellulose, la gomme xanthane, le carrageenane, la gomme guar.

16. Composition selon les revendications 7 ou 8 ou 9, **caractérisée en ce qu'**elle contient comme agent tensioactif une ou plusieurs substances choisies dans le groupe comprenant le laurylsulfate de sodium, l'alkylamidobétaïne, l'huile de ricin hydrogénée au PEG-40, le polysorbate-20.

17. Formulation selon les revendications 7 à 12, **caractérisée en ce qu'**elle contient comme charge aromatisante une ou plusieurs substances choisies dans le groupe comprenant les suivantes :
essence de menthe poivrée, essence de menthe verte, essence de sauge, essence de feuilles pour la peau, citrus, eucalyptus, sapin, anis, essences de girofle,
menthol, carbone, anéthol, salicylate de méthyle,
édulcorants - saccharinate de sodium, lactose, maltose, aspartame, cylamate de sodium.

18. Composition selon la revendication 7 ou 8, **caractérisé en ce qu'**elle contient comme conservateur une ou plusieurs substances choisies dans le groupe comprenant le méthylparabène, le propylparabène, le butylparabène ou leurs sels de sodium, le phénoxyéthanol, l'acide benzoïque, le benzoate de sodium.
